# FASCICULE DE BREVET EUROPEEN

(11) **EP 3 793 654 B1**
(45) Date de publication et mention de la délivrance du brevet: **20.04.2022**
(21) Numéro de dépôt: 19731323.2
(22) Date de dépôt: 16.05.2019
(51) Int. Cl.: A61M 15/08, B05B 11/00, A61M 11/00, A61M 15/00

(54) **DISPOSITIF DE DISTRIBUTION DE PRODUIT FLUIDE**
VORRICHTUNG ZUR AUSGABE EINES FLUIDPRODUKTS
DEVICE FOR DISPENSING A FLUID PRODUCT

(30) Priorité: 18.05.2018 FR 1854172
(43) Date de publication de la demande: 24.03.2021
(73) Titulaire: Aptar France SAS, 27110 Le Neubourg (FR)
(72) Inventeur: ADAM, Fabien, 27930 Aviron (FR); LEBEL, Baptiste, 27100 Val De Reuil (FR)
(74) Mandataire: CAPRI
(86) Numéro de dépôt international: PCT/FR2019/051119
(87) Numéro de publication internationale: WO 2019/220062

(56) Documents cités:
- WO-A1-2013/023258
- US-A1- 2003 041 859
- US-A1- 2005 268 915

## Description

La présente invention concerne un dispositif de distribution de produit fluide.

Plus particulièrement, la présente invention concerne un dispositif de distribution de produit fluide comportant une tête de distribution de produit fluide pour distribuer une ou deux dose(s) d'un produit fluide pharmaceutique. Dans ce type de dispositif unidose ou bidose, chaque dose de produit fluide, généralement liquide, est distribuée ou pulvérisée à travers un orifice de distribution lors d'un actionnement manuel de ce dispositif. Si le dispositif est un unidose, la totalité du produit fluide est distribuée en un seul actionnement. Si le dispositif est un bidose, le produit fluide est distribuée en deux actionnements successifs du dispositif.

Généralement, ce type de dispositif unidose ou bidose est utilisé pour une distribution nasale du produit fluide, et la tête de distribution comporte alors une extension axiale adaptée à pénétrer dans une narine d'un utilisateur lors de l'utilisation.

Notamment pour des raisons économiques, il peut être souhaitable de pouvoir utiliser le même dispositif aussi pour une distribution buccale. Ceci permettrait notamment d'éviter d'avoir à développer et réaliser un dispositif différent, avec tout ce que ça impose comme investissements en termes de développement et de chaine de production.

Or, une tête de distribution nasale est spécifiquement adaptée à la forme d'une narine, et utiliser un tel dispositif nasal pour une distribution buccale n'est pas adapté et donc pas optimal, notamment en terme d'ergonomie.

Le document WO2013023258 décrit un adaptateur pour un pulvérisateur latéral qui ne permet pas une distribution nasale. Cet adaptateur peut être un adaptateur pour la bouche seulement ou un adaptateur pour la bouche et le nez. Le document US2003041859 décrit un adaptateur pour inhalateur buccal qui se place autour de l'embout buccal de l'inhalateur. Le document US2005268915 décrit un embout nasal disposé autour d'un embout buccal d'un inhalateur.

La présente invention a pour but de fournir un dispositif de distribution de produit fluide qui ne reproduit pas les inconvénients susmentionnés.

Plus particulièrement, la présente invention a pour but de fournir un dispositif qui permette d'utiliser un dispositif nasal pour une distribution buccale.

La présente invention a également pour but de fournir un tel dispositif qui soit simple et peu coûteux à fabriquer et à assembler.

La présente invention a donc pour objet un dispositif de distribution de produit fluide, comportant un corps, un réservoir contenant du produit fluide, une tête de distribution nasale pourvue d'un orifice de distribution, et des moyens de distribution pour distribuer au moins une partie dudit produit fluide à travers ledit orifice de distribution lors d'un actionnement, ledit dispositif comportant un embout buccal disposé de manière amovible autour de ladite tête de distribution nasale.

Avantageusement, ladite tête de distribution nasale comporte un bossage radial saillant radialement vers l'extérieur, qui, à l'état assemblé dudit embout buccal, coopère avec une surface radialement interne dudit embout buccal.

Avantageusement, ledit embout buccal comporte une ouverture axiale, notamment circulaire, qui, à l'état assemblé dudit embout buccal, entoure ledit orifice de distribution de ladite tête de distribution nasale, pour permettre la distribution du produit fluide à travers ledit orifice de distribution.

Avantageusement, ledit embout buccal comporte une surface d'extrémité axiale sensiblement planaire qui inclut ladite ouverture axiale.

Avantageusement, ladite surface d'extrémité axiale est de forme oblongue, avec un bord radialement externe de forme ovale.

Avantageusement, ledit bord radialement externe de forme ovale a un grand axe de 15 à 20 mm, notamment 18 mm, et un petit axe de 10 à 15 mm, notamment 13,5 mm, ladite ouverture axiale ayant un rayon de 2 à 6 mm, notamment 3,5 mm.

Avantageusement, ledit embout buccal comporte une bride radiale qui, à l'état assemblé dudit embout buccal, épouse la forme d'une bride radiale dudit corps.

Avantageusement, ledit embout buccal comporte des moyens de positionnement.

Selon une première variante avantageuse, lesdits moyens de positionnement comportent une bride radiale permettant de limiter la profondeur d'insertion du dispositif entre les lèvres de l'utilisateur.

Selon une seconde variante avantageuse, lesdits moyens de positionnement comportent une languette axiale et radiale prévue à l'extrémité axiale dudit l'embout buccal.

Avantageusement, ledit réservoir comporte un bouchon qui obture ledit réservoir de manière étanche avant actionnement.

Avantageusement, ledit dispositif comporte une aiguille dont la pointe est adaptée à percer ledit bouchon lors de l'actionnement.

Avantageusement, ladite aiguille est fixe par rapport audit orifice de distribution.

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 est une vue schématique en section transversale d'un dispositif de distribution nasale de produit fluide selon l'art antérieur,
La figure 2 est une vue schématique de côté d'une tête de distribution buccale de produit fluide selon un premier mode de réalisation avantageux de la présente invention, avant assemblage sur le dispositif de distribution nasale de la figure 1,
Les figures 3 et 4 sont des vues similaires à celle de la figure 2, montrant la tête de distribution buccale après son assemblage sur le dispositif de distribution nasale de la figure 1, respectivement vu de dessus et de côté,
La figure 5 est une vue schématique en section transversale de la tête de distribution des figures 2 à 4,
La figure 6 est une vue de détail de la figure 5, montrant la fixation de l'organe buccal sur la tête nasale, et
Les figures 7 et 8 sont des vues similaires à celle de la figures 5, montrant deux variantes de réalisation avantageuses.

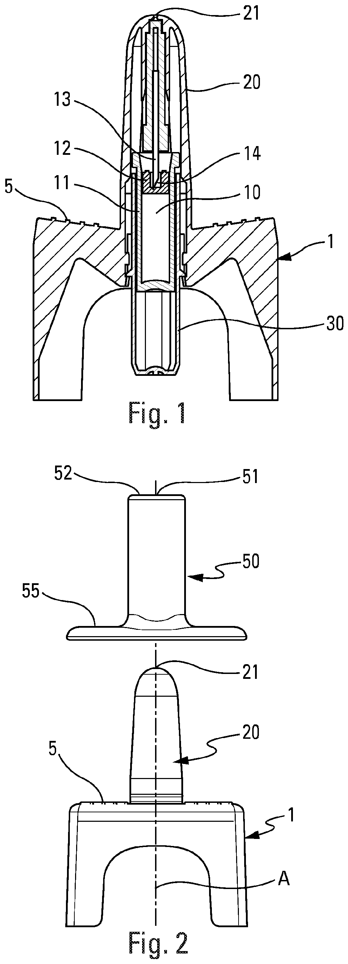

Les termes "proximal" et "distal" se réfèrent à l'orifice de distribution. Les termes "axial" et "radial" se réfèrent à l'axe central longitudinal A représenté sur les figures 2, 5, 7 et 8.

En référence à la figure 1, il est représenté un exemple d'un dispositif de distribution nasale de produit fluide auquel la présente invention s'applique. Dans cet exemple, il s'agit d'un dispositif unidose, dans lequel la totalité de la dose de produit fluide est distribuée en un seul actionnement. Il est à noter que la présente invention pourrait aussi s'adapter à d'autres types de dispositifs, tels que par exemple des dispositifs du type bidose, contenant deux doses de produit fluide à distribuer en deux actionnements successifs du dispositif.

Le dispositif comporte un corps 1, comportant une bride radiale 5 pour l'appui des doigts de l'utilisateur lors de l'actionnement.

Une tête de distribution nasale 20 s'étend axialement à partir de ladite bride radiale 5 et comporte un orifice de distribution 21, orienté axialement. Cet orifice de distribution 21 sert à distribuer une dose de produit fluide hors de ladite tête de distribution 20 lors de l'actionnement du dispositif par un utilisateur, cette distribution étant réalisée par des moyens de distribution. Avantageusement, la tête de distribution nasale 20 est réalisée d'une seule pièce monobloc avec ledit corps 1.

Le corps 1 reçoit un réservoir 10. Typiquement, le réservoir 10 peut être formé par un corps creux et borgne 11, comportant une seule ouverture fermée par un bouchon 12 et contenant une seule dose de produit fluide à distribuer un actionnement unique du dispositif.

La tête de distribution nasale 20 comporte des moyens de passage qui relient, en cours d'actionnement, le réservoir 10 à l'orifice de distribution 21. Ces moyens de passage comportent avantageusement une aiguille 13 creuse comportant une pointe 14 adaptée à transpercer le bouchon 12 au moment de l'actionnement. De préférence, cette aiguille 13 est fixe par rapport au corps 1 et notamment par rapport à l'orifice de distribution 21.

Des moyens d'actionnement 30 sont prévus pour permettre l'actionnement du dispositif. En l'occurrence, ces moyens d'actionnement 30 comportent un corps d'actionnement mobile par rapport au corps 1, ledit corps d'actionnement coopérant avec ledit réservoir 10 pour le déplacer axialement par rapport au corps 1 et donc par rapport à l'aiguille 13, en direction de l'orifice de distribution 21. Lors de ce déplacement, la pointe 14 de l'aiguille 13 perce le bouchon 12, puis celui-ci coulisse dans le réservoir 10 pour expulser le produit à travers ladite aiguille 13. Dans ce mode de réalisation, c'est le bouchon 12 qui forme les moyens de distribution. En variante, les moyens d'actionnement pourraient être formé par le fond du réservoir lui-même.

Dans un autre mode de réalisation, non représenté sur les dessins, le réservoir pourrait ne pas être formé par un corps creux borgne ne comportant qu'une ouverture, mais par un cylindre creux ouvert axialement des deux côtés. Ce cylindre serait alors fermé du côté proximal par un premier bouchon et du côté distal par un second bouchon, le volume défini entre lesdits deux bouchons contenant le produit fluide à distribuer. Lorsque l'utilisateur actionne le dispositif, il va comme décrit précédemment appuyer axialement sur le corps d'actionnement pour le faire coulisser axialement vers l'orifice de distribution. Ceci provoque le déplacement du second bouchon à l'intérieur du réservoir. Or, le produit fluide étant incompressible, ce déplacement du second bouchon va donc déplacer le premier bouchon en direction de l'aiguille, qui elle est fixe. Le premier bouchon va donc être percé par l'aiguille et le contenu du réservoir va être distribué à travers ladite aiguille par le second bouchon, qui agit alors en tant que piston. Dans ce mode de réalisation, c'est le second bouchon qui forme les moyens de distribution.

Comme évoqué précédemment, l'invention pourrait aussi s'appliquer à un dispositif du type bidose. Dans ce cas, le contenu du réservoir serait distribué en deux actionnements successifs. Le document WO2014147329 décrit un exemple de dispositif bidose.

Selon l'invention, il est prévu un embout buccal 50 adapté à être assemblé de manière amovible sur ladite tête de distribution nasale 20. Ainsi, le même dispositif de distribution peut être utilisé à la fois pour une distribution nasale, sans l'embout buccal 50, et pour une distribution buccale, après assemblage dudit embout buccal 50.

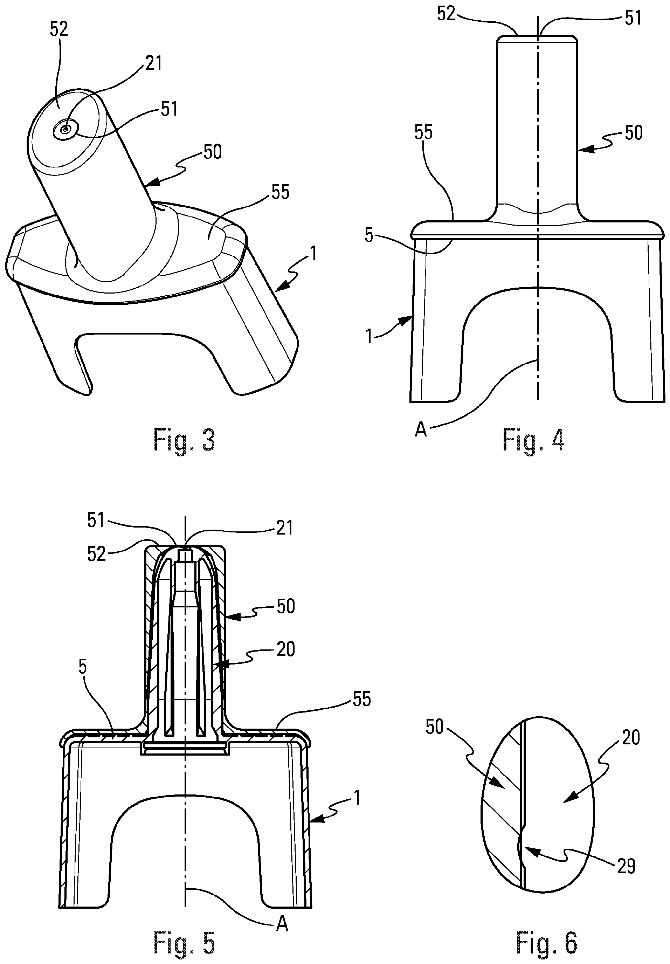

Avantageusement, l'embout buccal 50 vient s'emmancher sur la tête de distribution nasale 20. Comme représenté sur la figure 6, la tête de distribution nasale 20 peut comporter un bossage radial 29 saillant radialement vers l'extérieur, qui va coopérer avec une surface radialement interne de l'embout buccal 50, notamment pour renforcer son assemblage. Avantageusement, ce bossage 29 sert également à la fixation d'un capot de protection (non représenté) disposé autour de ladite tête de distribution nasale 20 lorsque le dispositif est stocké, pour protéger l'orifice de distribution 21.

L'embout buccal 50 comporte une ouverture axiale 51, avantageusement circulaire, qui, à l'état assemblé, va entourer l'orifice de distribution 21 de la tête de distribution nasale 20, pour permettre la distribution du produit fluide à travers ledit orifice de distribution 21.

L'embout buccal 50 comporte avantageusement une bride radiale 55 qui va épouser la forme de la bride radiale 5 du corps 1. Ceci permet notamment de parfaitement positionner ledit embout buccal autour de ladite tête de distribution nasale 20.

Avantageusement, l'embout buccal 50 comporte une surface d'extrémité axiale 52 sensiblement planaire et qui inclut ladite ouverture axiale 51.

Comme visible sur la figure 3, cette surface d'extrémité axiale 52 peut être de forme oblongue, avec par exemple un bord radialement externe de forme ovale. Avantageusement, ledit ovale a un grand axe de 15 à 20 mm, de préférence 18 mm, et un petit axe de 10 à 15 mm, de préférence 13,5 mm. Dans cette configuration, l'ouverture axiale 51 a avantageusement un rayon de 2 à 6 mm, de préférence 3,5 mm. Cette mise en œuvre favorise un bon positionnement de l'embout buccal 50 entre les lèvres de l'utilisateur lors de l'actionnement, et permet notamment d'éviter que les mâchoires soient fermées, avec les dents empêchant alors la bonne distribution du produit fluide dans la bouche. De plus, un tel embout buccal améliore l'ergonomie et le confort de l'utilisateur en cas de distribution buccale, et permet d'éviter que l'utilisateur ne se trompe de voie d'administration, les dimensions dudit embout buccal étant incompatibles avec une administration nasale.

En variante, ledit bord externe de cette surface d'extrémité axiale 52 pourrait aussi être circulaire, et plus généralement toute forme appropriée est envisageable.

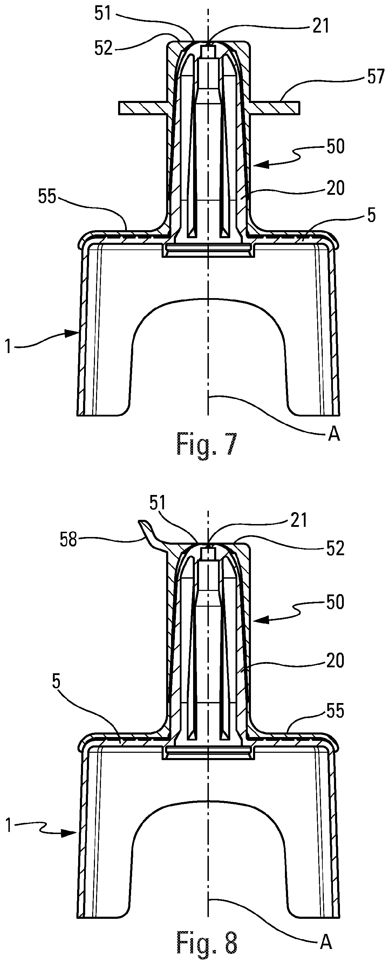

Les figures 7 et 8 illustrent deux variante de réalisation, dans lesquelles l'embout buccal 50 comporte des moyens de positionnement 57, 58, utiles lors de l'actionnement. Dans l'exemple de la figure 7, il est prévu une bride radiale externe 57 permettant notamment de limiter la profondeur d'insertion du dispositif entre les lèvres de l'utilisateur. Dans l'exemple de la figure 8, une languette axiale et radiale 58 est prévue à l'extrémité axiale de l'embout buccal 50. Cette languette 58 permet en cas de distribution sublinguale de former un support pour la langue de l'utilisateur, notamment pour éviter que celle-ci ne vienne obstruer l'orifice de distribution 21 lors de l'actionnement.

Dans une autre variante (non représentée), l'embout buccal pourrait transformer une distribution axiale de la tête de distribution nasale en une distribution radiale, auquel cas l'ouverture de l'embout buccal ne serait plus axiale mais radiale, avec de préférence des moyens de pulvérisation appropriés, tel qu'un gicleur.

Bien entendu, d'autres variantes de réalisation sont également envisageables, sans sortir du cadre de la présente invention, tel que défini par les revendications annexées.

## Revendications

1. Dispositif de distribution de produit fluide, comportant un corps (1), un réservoir (10) contenant du produit fluide, une tête de distribution nasale (20) pourvue d'un orifice de distribution (21), et des moyens de distribution (12) pour distribuer au moins une partie dudit produit fluide à travers ledit orifice de distribution (21) lors d'un actionnement, **caractérisé en ce que** ledit dispositif comporte un embout buccal (50) disposé de manière amovible autour de ladite tête de distribution nasale (20).

2. Dispositif selon la revendication 1, dans lequel ladite tête de distribution nasale (20) comporte un bossage radial (29) saillant radialement vers l'extérieur, qui, à l'état assemblé dudit embout buccal (50), coopère avec une surface radialement interne dudit embout buccal (50).

3. Dispositif selon la revendication 1 ou 2, dans lequel ledit embout buccal (50) comporte une ouverture axiale (51), notamment circulaire, qui, à l'état assemblé dudit embout buccal (50), entoure ledit orifice de distribution (21) de ladite tête de distribution nasale (20), pour permettre la distribution du produit fluide à travers ledit orifice de distribution (21).

4. Dispositif selon la revendication 3, dans lequel ledit embout buccal (50) comporte une surface d'extrémité axiale (52) sensiblement planaire qui inclut ladite ouverture axiale (51).

5. Dispositif selon la revendication 4, dans lequel ladite surface d'extrémité axiale (52) est de forme oblongue, avec un bord radialement externe de forme ovale.

6. Dispositif selon la revendication 5, dans lequel ledit bord radialement externe de forme ovale a un grand axe de 15 à 20 mm, notamment 18 mm, et un petit axe de 10 à 15 mm, notamment 13,5 mm, ladite ouverture axiale (51) ayant un rayon de 2 à 6 mm, notamment 3,5 mm.

7. Dispositif selon l'une quelconque des revendications précédentes, dans lequel ledit embout buccal (50) comporte une bride radiale (55) qui, à l'état assemblé dudit embout buccal (50), épouse la forme d'une bride radiale (5) dudit corps (1).

8. Dispositif selon l'une quelconque des revendications précédentes, dans lequel ledit embout buccal (50) comporte des moyens de positionnement (57, 58).

9. Dispositif selon la revendication 8, dans lequel lesdits moyens de positionnement comportent une bride radiale (57) permettant de limiter la profondeur d'insertion du dispositif entre les lèvres de l'utilisateur.

10. Dispositif selon la revendication 8, dans lequel lesdits moyens de positionnement comportent une languette axiale et radiale (58) prévue à l'extrémité axiale dudit l'embout buccal (50).

11. Dispositif selon l'une quelconque des revendications précédentes, dans lequel ledit réservoir (10) comporte un bouchon (12) qui obture ledit réservoir (10) de manière étanche avant actionnement.

12. Dispositif selon la revendication 11, comportant une aiguille (13) dont la pointe (14) est adaptée à percer ledit bouchon (12) lors de l'actionnement.

13. Dispositif selon la revendication 12, dans lequel ladite aiguille (13) est fixe par rapport audit orifice de distribution (21).

## Patentansprüche

1. Vorrichtung zur Abgabe eines Flüssigprodukts, umfassend einen Körper (1), einen Vorratsbehälter (10), der ein Flüssigprodukt enthält, einen Nasalabgabekopf (20), der mit einer Abgabeöffnung (21) versehen ist, und Abgabemittel (12), um bei einer Betätigung mindestens einen Teil des Flüssigprodukts über die Abgabeöffnung (21) abzugeben, **dadurch gekennzeichnet, dass** die Vorrichtung ein Mundstück (50) umfasst, das abnehmbar um den Nasalabgabekopf (20) herum angeordnet ist.

2. Vorrichtung nach Anspruch 1, bei der der Nasalabgabekopf (20) einen radial nach außen vorstehenden radialen Vorsprung (29) aufweist, der bei angebrachtem Mundstück (50) mit einer radial inneren Fläche des Mundstücks (50) zusammenwirkt.

3. Vorrichtung nach Anspruch 1 oder 2, bei der das Mundstück (50) eine axiale, insbesondere kreisförmige Öffnung (51) aufweist, die bei angebrachtem Mundstück (50) die Abgabeöffnung (21) des Nasalabgabekopfs (20) umgibt, um die Abgabe des Flüssigprodukts über die Abgabeöffnung (21) zu ermöglichen.

4. Vorrichtung nach Anspruch 3, bei der das Mundstück (50) eine im Wesentlichen ebene axiale Endoberfläche (52) aufweist, die die axiale Öffnung (51) einschließt.

5. Vorrichtung nach Anspruch 4, bei der die axiale Endoberfläche (52) länglich geformt ist, mit einem radial äußeren Rand von ovaler Form.

6. Vorrichtung nach Anspruch 5, bei der der radial äußere Rand von ovaler Form eine längere Achse von 15 mm bis 20 mm, insbesondere von 18 mm, und eine kürzere Achse von 10 mm bis 15 mm, insbesondere von 13,5 mm, hat, wobei der Radius der axialen Öffnung (51) zwischen 2 mm und 6 mm, insbesondere 3,5 mm, beträgt.

7. Vorrichtung nach einem der vorhergehenden Ansprüche, bei der das Mundstück (50) einen radialen Flansch (55) aufweist, der bei montiertem Mundstück (50) die Form eines radialen Flansches (5) des Körpers (1) annimmt.

8. Vorrichtung nach einem der vorhergehenden Ansprüche, bei der das Mundstück (50) Positioniermittel (57, 58) umfasst.

9. Vorrichtung nach Anspruch 8, bei der die Positioniermittel einen radialen Flansch (57) umfassen, mit dem sich die Einführtiefe der Vorrichtung zwischen den Lippen des Benutzers begrenzen lässt.

10. Vorrichtung nach Anspruch 8, bei der die Positioniermittel eine axiale und radiale Zunge (58) umfassen, die am axialen Ende des Mundstücks (50) vorgesehen ist.

11. Vorrichtung nach einem der vorhergehenden Ansprüche, bei der der Vorratsbehälter (10) einen Stopfen (12) aufweist, der vor Betätigung den Vorratsbehälter (10) dicht verschließt.

12. Vorrichtung nach Anspruch 11 mit einer Nadel (13), deren Spitze (14) dazu geeignet ist, bei Betätigung den Stopfen (12) zu durchstechen.

13. Vorrichtung nach Anspruch 12, bei der die Nadel (13) bezüglich der Abgabeöffnung (21) feststehend ist.

## Claims

1. A device for dispensing a fluid product, including a body (1), a reservoir (10) containing fluid product, a nasal dispensing head (20) provided with a dispensing orifice (21) and dispensing means (12) for dispensing at least a portion of said fluid product through said dispensing orifice (21) during an actuation, **characterized in that** said device includes a mouthpiece (50) arranged removably around said nasal dispensing head (20).

2. The device according to claim 1, wherein said nasal distribution head (20) includes a radial boss (29) protruding radially outward which, in the assembled state of said mouthpiece (50), cooperates with a radially internal surface of said mouthpiece (50).

3. The device according to claim 1 or 2, wherein said mouthpiece (50) includes an axis opening (51), circular in particular which, in the assembled state of said mouthpiece (50), surrounds said dispensing orifice (21) of said nasal dispensing head (20) to allow the dispensing of the fluid product through said dispensing orifice (21).

4. The device according to claim 3, wherein said mouthpiece (50) includes a substantially flat axial end surface (52) which includes said axial opening (51).

5. The device according to claim 4, wherein said axial end surface (52) has an oblong shape, with an oval-shaped radially external edge.

6. The device according to claim 5, wherein said radially external oval-shaped edge has a major axis of 15 to 20 mm, particularly 18 mm, and a minor axis of 10 to 15 mm, particularly 13.5 mm, said axial opening (51) having a radius of 2 to 6 mm, particularly 3.5 mm.

7. The device according to any one of the preceding claims, wherein said mouthpiece (50) includes a radial flange (55) which, in the assembled state of said mouthpiece (50), matches the shape of a radial flange (5) of said body (1).

8. The device according to any one of the preceding claims, wherein said mouthpiece (50) includes positioning means (57, 58) .

9. The device according to claim 8, wherein said positioning means include a radial flange (57) allowing limiting the insertion depth of the device between the lips of the user.

10. The device according to claim 8, wherein said positioning means include an axial and radial strip (58) provided at the axial end of said mouthpiece (50).

11. The device according to any one of the preceding claims, wherein said reservoir (10) includes a stopper (12) which blocks said reservoir (10) in a sealed manner prior to actuation.

12. The device according to claim 11, including a needle (13), the point (14) of which is suited for piercing said stopper (12) during actuation.

13. The device according to claim 12, wherein said needle (13) is fixed relative to said dispensing orifice (21).
